# EUROPÄISCHE PATENTSCHRIFT

(11) **EP 0 381 718 B1**
(45) Veröffentlichungstag und Bekanntmachung des Hinweises auf die Patenterteilung: **28.09.1994**
(21) Anmeldenummer: 89907748.1
(22) Anmeldetag: 10.07.1989
(51) Int. Cl.: A61F 2/46

(54) **MARKHÖHLENABDICHTVORRICHTUNG**
SEALING DEVICE FOR THE MEDULLARY CAVITY
DISPOSITIF D'ETANCHEIFICATION DE CAVITES MEDULLAIRES

(30) Priorität: 08.07.1988 DE 3823287
(43) Veröffentlichungstag der Anmeldung: 16.08.1990
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: EP8900794
(87) Internationale Veröffentlichungsnummer: WO9000375

(56) Entgegenhaltungen:
- EP-A- 0 073 604
- EP-A- 0 170 120
- EP-A- 0 320 138
- CH-A- 639 549
- US-A- 4 274 163
- US-A- 4 338 925
- US-A- 4 815 454

## Beschreibung

Die Erfindung betrifft eine Vorrichtung zum Abdichten der Markhöhle eines Knochens bei der Applikation von Knochenzement.

In der Gelenkersatzchirurgie werden die meisten Implantate mit Hilfe von sogenanntem Knochenzement im knöchernen Lager fixiert. Diese Knochenzemente sind in aller Regel Polymethylmethacrylate oder verwandte Verbindungen. Um das Eindringen des Knochenzements in die Knochenmarkswaben zu verbessern, wurde versucht, das Knochenlager unter Druck auszuwaschen und den Knochenzement unter Druck einzupressen. Bei dieser Technik, die auch als "high pressurizing technique" bekannt ist, wurden vor allem Knochenzemente mit niedriger Viskosität verwendet.

Bei dieser bekannten Technik gab es jedoch eine Reihe von Zwischenfällen mit tödlichem Ausgang. Sowohl tierexperimentelle Studien als auch klinische Untersuchungen zeigten, daß die intramedulläre Druckerhöhung zu reflektorischen Herzstillständen führen kann, daß aber auch tödliche Fett- und Knochenmarksembolien auftreten können. Zudem gelang es nicht, das knöcherne Bett auf diese Weise bluttrocken zu halten, vielmehr strömte das Blut je nach Höhe des Blutdruckes in das knöcherne Bett ein und vermischte sich mit dem Knochenzement, was dessen Materialeigenschaften äußerst ungünstig beeinflußte.

Es wurde auch bereits versucht, die mechanische Festigkeit der in den Kliniken angewandten Knochenzemente durch Vakuummischen und Vorkomprimierung des Knochenzements zu verbessern. Es ist jedoch wesentlich, daß die einmal erreichte Materialverfestigung des Knochenzements auch ohne Gefahr für den Patienten in das knöcherne Bett übertragen werden kann.

Es wurde deshalb eine Methode entwickelt, um das Knochenbett bluttrocken zu halten und artefaktfrei mit Zement aufzufüllen, ohne dabei den Patienten durch temporäre Erhöhung des intramedulären Druckes zu gefährden. Bei dieser Methode wird der unter Vakuum angemischte und vorkomprimierte Knochenzement mit einer Knochenzementpistole oder -presse, wie sie beispielsweise in der EP-A-170 120 oder US-A-4 671 263 beschrieben ist, in die Markhöhle appliziert, während gleichzeitig distal mittels einer kanülierten Knochenschraube ein Vakuum angelegt wird. Auf diese Weise wird der Knochenzement in die Tiefe der Markhöhle eingesaugt, während gleichzeitig Blut, Fett und Knochenmark aus dem Knochenkanal abgesaugt werden. Nach Einbringen der Prothesenkomponente wird die distale Vakuumleitung abgeklemmt und proximal ebenfalls über eine kanülierte Knochenschraube ein Vakuum angelegt, so daß das Vakuum in der Methaphyse bis zur Aushärtung des Knochenzements wirksam bleibt. Durch diese Methode, die durch Vakuumauffüllung der Markhöhle bei gleichzeitiger Drainage gekennzeichnet ist, wird eine vollständige und artefaktfreie Auffüllung des Knochenzements um die Prothese und ein tiefes Eindringen des Knochenzements in die Spongiosa ermöglicht.

Um den erforderlichen Unterdruck in der Markhöhle aufrecht zu erhalten und ein Herausdringen des Knochenzements zu verhindern, ist es wesentlich, die Markhöhle während der Applikation des Knochenzements soweit als möglich abzudichten.

Dies wurde bisher mit einer um die Spitze des Behälters oder der Kartusche, aus der der Knochenzement appliziert wird, gelegten Manschette versucht.

EP-A-0 073 604 betrifft die Zementapplikation unter Druck ("high pressurizing technique"). Gemäß Fig. 2 weist die Abdichtvorrichtung gemäß EP-A-0 073 604 einen flüssigkeitsgefällten Ballon aus einem dehnbaren Material sowie ein Instrument mit einem Handgriff und einer flachen Platte auf. Mittels dieses Instruments soll Druck auf die Flüssigkeit innerhalb des Ballons ausgeübt werden und dadurch die Markhöhle am oberen Rand entlang ihrer Innenwand abgedichtet werden. Außerdem soll damit Druck auf den Knochenzement ausgeübt werden.

Die Vorrichtung gemäß EP-A-0 073 604 ergibt nur bei Anwendung einer Druckapplikationstechnik eine Abdichtung des Innenrandes der Markhöhle, nicht jedoch bei der Vakuum-Applikation.

Der Erfindung liegt die Aufgabe zugrunde, eine Vorrichtung zum Abdichten der Markhöhle eines Knochens bei der Applikation von Knochenzement bereitzustellen, mit dem die Markhöhle in dem Bereich, in dem der Knochenzement appliziert wird, praktisch vakuumdicht abgedichtet werden kann.

Diese Aufgabe wird durch die erfindungsgemäße Vorrichtung gemäß den Patentansprüchen gelöst.

Gegenstand der Erfindung ist somit eine Art Markhöhlensiegel bzw. eine Markhöhlenversiegelung oder -abdichtung.

Die Erfindung geht dabei von dem Grundgedanken aus, eine Vorrichtung mit einem flexiblen oder weichen Unterteil vorzusehen, das als Dichtung wirkt und sich unter Ausübung von mechanischem Druck wie ein Dichtring um die Öffnung der Markhöhle legt, und das flexible Unterteil durch ein hartes oder starres Oberteil um die Markhöhle herum an den Knochen anzupressen, beispielsweise an die Resektionsebene. Das Unterteil wirkt somit praktisch als elastisches Zwischenstück und das Oberteil als Schild, mit dem das Zwischenstück insbesondere in der Anfangsphase der Evakuierung angepreßt wird, bis sich ein Unterdruck in der Markhöhle aufgebaut hat, durch den das Unterteil abdichtend am Knochen festgehalten wird.

Vorzugsweise wird die Abdichtvorrichtung direkt mittels der Spitze des Behälters oder der Kartusche angepreßt, die den Knochenzement enthält. Zu diesem Zweck ist die Form der Innenseite des Unterteils und/oder des Oberteils der Abdichtvorrichtung vorzugsweise an die Form der Kartuschenspitze angepaßt. Falls die Kartuschenspitze konisch zuläuft, ist die Innenseite der Abdichtvorrichtung, vorzugsweise des elastischen Unterteils, ebenfalls zumindest teilweise als konische Auflagefläche ausgebildet. Wenn die Kartusche zylindrisch ist, ist auch die Auflagefläche vorzugsweise zylindrisch, mit demselben oder einem geringfügig größeren Durchmesser, so daß eine formschlüssige Aufnahme der Kartusche gewährleistet ist. Wenn die Kartusche gegen die Abdichtvorrichtung gepreßt wird, tritt eine praktisch vakuumdichte Abdichtung einerseits zwischen der Kartuschenspitze und der Auflagefläche der Abdichtvorrichtung und andererseits zwischen der unteren Dichtfläche der Abdichtvorrichtung und dem Knochen um die Öffnung des Markkanals herum ein, so daß sich ein Unterdruck in der Markhöhle aufbauen kann und der applizierte Knochenzement nicht wieder aus der Markhöhle herausgedrückt werden kann. Die Kartuschenspitze paßt in die Aufnahme aus Silikongummi. Falls die Kartuschenspitze in dem harten Oberteil aufgenommen wird, kann zur Abdichtung dazwischen Vakuumfett oder Dichtfett verwendet werden.

Falls die Auflagefläche des Unterteils auf dem Knochen als ebene Fläche ausgebildet ist, wie beispielsweise die Resektionsebene des Femurs bei einer Hüftgelenksoperation, kann die untere Fläche des Unterteils ebenfalls als plane Fläche ausgebildet sein. Vorzugsweise ist die Fläche jedoch konvex und wird erst beim Andrücken abgeplattet. Auf diese Weise ist eine ausgezeichnete Abdichtung gewährleistet.

Um eine optimale Ausfüllung der Spongiosaräume, die nach oben offene Trichtersysteme darstellen, zu gewährleisten, weist die Unterseite des Unterteils eine Ausnehmung oder Aussparung auf. Der Umfang der Ausnehmung ist so bemessen, daß ein möglichst großer Teil der Resektionsebene um die Markhöhlenöffnung herum freiliegt, so daß ein großflächiges Auffüllen der Spongiosa gewährleistet ist.

Bei der Anwendung im Azetabulum bzw. der Sichelpfanne des Beckens ist das Unterteil vorzugsweise zumindest teilweise als Wulst oder Dichtring ausgebildet, der so bemessen ist, daß er nicht am Rand der Pfanne, sondern im tragenden Dach aufsitzt und abdichtet. Besonders bevorzugt weist das Unterteil oberhalb des abdichtenden Wulstes eine halbkugelförmige Halbschale auf, die formschlüssig mit einem ebenfalls halbkugelförmigen Teil des Oberteils in Verbindung steht. Das Unterteil ist dabei in Form eines geschlossenen Inlays ausgebildet. Hierdurch ist sichergestellt, daß eventuell nach oben herausgedrückter Knochenzement nur mit dem Material des Unterteils, nicht jedoch mit dem Oberteil in Berührung kommt.

Das Unterteil kann entweder das Oberteil in einem Schlitz aufnehmen und/oder umgekehrt in einer Rinne des Oberteils liegen.

Die erfindungsgemäße Vorrichtung ist grundsätzlich bei allen Gelenkersatzoperationen anwendbar, wobei ihre Ausbildung im einzelnen von der speziellen Anwendung abhängt.

Vorzugsweise sollte das Unterteil an seiner Dichtfläche im wesentlichen ringförmig oder wulstförmig oder bikonvex sein. Durch die Rundung des Unterteils wird ein Verbiegen oder Faltenwurf verhindert, der bei einem flach oder als Scheibe ausgebildeten Unterteil auftreten kann; in diesem Falle ist keine saubere Anlage an der Resektionsebene gewährleistet, und durch die Undichtigkeit kann sich kein ausreichender Unterdruck in der Markhöhle aufbauen.

Die Oberseite des flexiblen Unterteils und die Unterseite des starren Oberteils sind möglichst formschlüssig aneinander angepaßt, so daß der auf das starre Oberteil ausgeübte Druck auf das Unterteil übertragen und dieses an den Knochen um die Markhöhle herum abdichtend angepreßt werden kann. Vorzugsweise weisen das Unterteil und das Oberteil eine möglichst große Kontaktfläche auf. Diese Kontaktfläche kann beispielsweise etwa die Form einer sich nach oben verjüngenden Halbkugelfläche aufweisen. Falls das Unterteil ringförmig ausgebildet ist, weist die Unterseite des Oberteils vorzugsweise eine ringförmige Nut zur formschlüssigen Aufnahme des Unterteils auf.

Vorzugsweise sind Einrichtungen vorgesehen, um ein Verdrehen von Unterteil und Oberteil gegeneinander zu verhindern, beispielsweise mehrere Zapfen, die vom Unterteil oder Oberteil vorspringen und in Aussparungen des anderen Teils hineinpassen. Das weiche Unterteil kann auch als komplettes Inlay an die Form des Oberteils angepaßt sein, beispielsweise in Form von zwei Halbschalen mit Zapfen und korrespondierenden Ausnehmungen zur mechanischen Fixierung. Die beiden Teile können auch fest miteinander verbunden, beispielsweise miteinander verklebt sein.

Vorzugsweise besteht das elastische Unterteil aus Silikongummi oder ähnlichen Materialien, wie sie in der Vakuumtechnik verwendet werden. Das Oberteil besteht vorzugsweise aus einem Metall, wie Aluminium, Stahl oder Titan, oder aus einem harten Kunststoff, wie Teflon®, TPX® oder einem anderen sterilisierbaren Kunststoff.

Die Erfindung wird nachstehend anhand der Zeichnung näher erläutert. Es zeigen:
- Fig. 1: einen Querschnitt entlang Linie I-I von Fig. 2 einer erfindungsgemäßen Vorrichtung zur Anwendung bei der Abdichtung der Markhöhle des Femurs,
- Fig. 2: eine Draufsicht der Vorrichtung gemäß Fig. 1,
- Fig. 3: einen Querschnitt entlang Linie III-III von Fig. 4 einer erfindungsgemäßen Vorrichtung zur Anwendung im Azetabulum des Beckens,
- Fig. 4: eine schematische Draufsicht der Vorrichtung gemäß Fig. 3, und
- Fig. 5: eine weitere erfindungsgemäße Vorrichtung zur Anwendung im Azetabulum des Beckens.

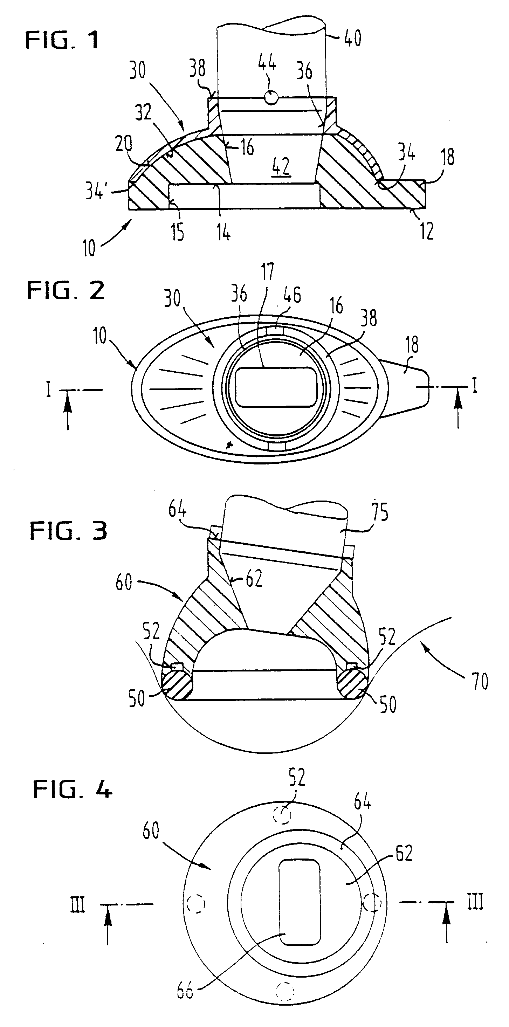

Die Abdichtvorrichtung gemäß Fig. 1 weist ein flexibles, weiches Unterteil 10 und ein hartes, starres Oberteil 30 auf. Das Unterteil 10 weist eine ebene untere Fläche 12 auf, die bei der Anwendung auf der Resektionsebene aufliegt. Wie vorstehend erläutert, kann die untere Abdichtfläche auch wulstförmig oder konvex ausgebildet sein, um eine sichere Abdichtung zu gewährleisten. Um eine zentrale Öffnung herum ist eine Ausnehmung 14 vorgesehen, deren umlaufender Rand 15 etwa oval oder elliptisch ist. Diese Ausnehmung 14 ist derart bemessen, daß die Spongiosawaben um die Markhöhlenöffnung herum freiliegen und die untere Fläche 12 lediglich an einem äußeren Rand von einigen Millimetern Breite auf dem Knochen aufliegt. Auf diese Weise können die freiliegenden Spongiosawaben mit Knochenzement gefüllt werden. Im Innern weist das Unterteil 10 eine durchgehende, nach unten konisch zulaufende Öffnung mit einer konischen Anlage- oder Auflagefläche 16 auf, die die konische Spitze der Knochenzementkartusche umfaßt. Wenn die Kartusche gegen die Abdichtvorrichtung gedrückt wird, dichtet die Anlagefläche 16 vakuumdicht gegen die Kartuschenspitze ab.

Zur Vergrößerung der Dichtfläche und Verbesserung der Stabilität weist das Unterteil 10 einen Ansatz 18 auf. Die Oberseite 20 des Unterteils 12 ist wulstförmig oder kugelig gewölbt. Hierdurch wird beim Anpressen des Oberteils 30 die Stabilität erhöht.

Die Unterseite 32 des Oberteils 30 ist ebenfalls gewölbt und liegt bündig auf der Oberseite 20 des Unterteils 10 auf. Der umlaufende untere Rand 34, 34' des Oberteils 30 liegt ebenfalls schlüssig auf einem Absatz des Unterteils 10 auf. An der Innenseite weist das Oberteil 30 oben einen geraden, zylindrischen Ansatz und darunter ebenfalls eine konische Öffnung 36 auf. An der Oberseite ist ein Rand 38 vorgesehen.

In Fig. 1 ist ebenfalls eine Knochenzementkartusche 40 mit konischer Spitze 42 und Zapfen 44 eingezeichnet. Bei der Applikation wird die Kartusche 40 nach unten gedrückt. Die Zapfen 44 stoßen gegen den oberen Rand 38 des Oberteils 30 und pressen das Oberteil 30 nach unten gegen das Unterteil 10. Wenn genügend Druck ausgeübt wird, erfolgt einerseits eine Abdichtung zwischen der konischen Spitze 42 und der Anlagefläche 16 und andererseits zwischen der unteren Fläche 12 und dem Knochen. Auf diese Weise wird die Markhöhle praktisch vakuumdicht abgedichtet und ein Herausdringen des applizierten Knochenzements verhindert. Bei Applikation des Knochenzements beispielsweise proximal in die Markhöhle des Femurs wird die Markhöhle distal mittels einer kanülierten Knochenschraube abgesaugt. Durch die proximale Abdichtung kann sich, während der Knochenzement appliziert wird, ein Unterdruck bzw. ein Vakuum in der Markhöhle ausbilden. Auf diese Weise kann die Vakuumauffüllung mit gleichzeitiger Drainage problemlos durchgeführt werden.

Fig. 2 zeigt zur weiteren Erläuterung eine Draufsicht der Vorrichtung gemäß Fig. 1 ohne Knochenzementkartusche. In Fig. 2 ist die konische Anlagefläche 16 des Unterteils 10 mit der ovalen Öffnung 17 erkennbar, deren Form in etwa der Form der Öffnung der Kartuschenspitze entspricht. Der runde obere Rand 38 umfaßt die zylindrische Kartusche 40. Der Konus 36 ist ebenfalls der Form der Kartuschenspitze angepaßt. Im Rand 38 sind zwei Ausnehmungen oder Vertiefungen 46 zur bündigen Aufnahme der Zapfen 44 der Kartusche 40 vorgesehen, wodurch ein stabiles Andrücken der Kartusche 40 gewährleistet ist.

Die Abdichtvorrichtung für das Azetabulum des Beckens gemäß Fig. 3 weist ein flexibles, weiches Unterteil 50 und ein starres Oberteil 60 auf. Das Unterteil 50 ist ringförmig ausgebildet und weist Zapfen 52 auf, die in Ausnehmungen des Oberteils 60 eingreifen, um eine Verschiebung von Unterteil und Oberteil gegeneinander zu verhindern. Das Oberteil 60 ist in Form eines innen hohlen Körpers ausgebildet, wobei seine Innenseite 62 von oben her konisch zuläuft, um die Spitze einer Kartusche 75 aufzunehmen. Zur besseren Abdichtung zwischen der konischen Anlagefläche 62 und der Kartuschenspitze kann auf der Anlagefläche 62 noch eine elastische Zwischenschicht ausgebildet sein, die ebenfalls konusförmig ist. Oben weist das Oberteil 60 einen runden, umlaufenden Rand 64 auf.

In Fig. 3 ist auch die Anwendung der Abdichtvorrichtung in einem Azetabulum 70 schematisch dargestellt. Der Knochenzement befindet sich in der Kartusche 75, die der Kartusche 40 gemäß Fig. 1 entspricht, wobei die Kartusche von Fig. 3 gegenüber der von Fig. 1 um 90° gedreht ist. Der Operateur preßt die Kartusche 75 mit ihrer konischen Spitze gegen die innere Anlagefläche 62 der Abdichtvorrichtung und drückt diese nach unten. Dabei wird das als Dichtring ausgebildete Unterteil 50 gegen das tragende Dach des Azetabulums gedrückt und dichtet dort vakuumdicht ab. Danach wird der Knochenzement aus der Kartusche 75 heraus dosiert appliziert, während gleichzeitig eine Drainage angelegt und der Knochenzement durch den entstehenden Unterdruck in die Tiefe der Spangiosawaben eingesaugt wird.

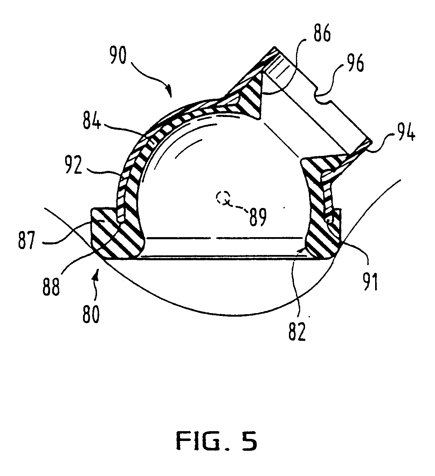

In der Ansicht von Fig. 4, in der die Kartusche weggelassen ist, ist ersichtlich, daß die ovale Öffnung der Innenseite 62 des Oberteils 60, die die Kartuschenspitze aufnimmt, und der Rand 64 etwas asymmetrisch zum abdichtenden Unterteil 50 sind. In Fig. 4 sind ebenfalls schematisch die vier Zapfen 52 des Unterteils 50 eingezeichnet.

Fig. 5 zeigt eine ähnliche Abdichtvorrichtung für das Azetabulum des Beckens wie Fig. 3. Bei der Abdichtvorrichtung gemäß Fig. 5 weist das flexible, weiche Unterteil 80 neben dem Ring oder Wulst 82, der dem Unterteil 50 gemäß Fig. 3 ähnelt, noch eine etwa halbkugelförmige Schale 84 und ein Aufnahmestück 86 für die Kartuschenspitze auf. Das ring- oder wulstförmige Teil 82 kann einen Ansatz 87 aufweisen, der der besseren Stabilität und besseren Abdichtung durch Anpassung an die Form des Azetabulums dient. Zwischen dem ringförmigen Teil 82 und der Halbschale 84 ist eine umlaufende Nut 88 vorgesehen. In die Nut 88 paßt der untere Rand 91 eines starren Oberteils 90. Um ein relatives Verdrehen von Unterteil 80 und Oberteil 90 zu verhindern, sind mehrere Zapfen 89 am Unterteil 80 vorgesehen, die in entsprechende Ausnehmungen im Oberteil 90 eingreifen. Das Oberteil 90 weist im wesentlichen eine halbkugelförmige Schale 92 und einen Stutzen 94 auf, welche die halbkugelförmige Schale 84 des Unterteils bzw. das Aufnahmeteil 86 des Oberteils formschlüssig umschließen.

Wie aus Fig. 5 ersichtlich ist, ist das Aufnahmeteil für die Knochenzementkartusche, bezogen auf die durch den unteren Abdichtring 82 ausgebildete Ebene, seitlich unter einem Winkel von etwa 30-60°, vorzugsweise etwa 45°, angesetzt. Dies erleichtert die Zugänglichkeit und das Ansetzen der Kartusche während des Operationsvorgangs.

An seinem oberen Rand weist das Oberteil 90 mehrere, vorzugsweise zwei Ausnehmungen 96 zur Aufnahme von an der Kartusche angeordneten Zapfen auf.

Die Anwendung der Abdichtvorrichtung gemäß Fig. 5 entspricht derjenigen gemäß Fig. 3. Die (in Figur 5 nicht dargestellte) Kartusche wird vom Operateur mit ihrer konischen Spitze gegen die konische Fläche 86 gedrückt, wobei der obere Rand des Oberteils bzw. die Ausnehmungen 96 als Anschlag für die Kartusche bzw. deren Zapfen dienen. Dadurch wird der Ring 82 des Unterteils 80 gegen das tragende Dach des Azetabulums gedrückt und dichtet dort vakuumdicht ab, während der Knochenzement aus der Kartusche heraus appliziert wird.

## Patentansprüche

1. Vorrichtung zum Abdichten der Markhöhle eines Knochens bei der Vakuum-Applikation von Knochenzement, mit einem elastischen Unterteil (10; 50; 80) zum Aufliegen auf dem Knochen und zum praktisch vakuumdichten Kontakt mit dem Knochen und mit einem starren Oberteil (30; 60; 90) zum Anpressen des elastischen Unterteils an den Knochen wobei das Oberteil schalenförmig nach oben gewölbt ist, und wobei Unter- und Oberteil eine gemeinsame Öffnung zum Applizieren des Knochenzements aufweisen.

2. Vorrichtung nach Anspruch 1, dadurch gekennzeichnet, daß das Unterteil (10; 50; 80) und das Oberteil (30; 60; 90) im wesentlichen etwa hohlzylindrisch, ringförmig halbkugelförmig oder als Ellipsoid ausgebildet sind, und das Unterteil und/oder das Oberteil an ihrer Innenseite eine Auflagefläche (16, 62; 86) zur formschlüssigen Aufnahme der Spitze eines Behälters (40; 75) aufweisen, aus dem heraus der Knochenzement appliziert wird.

3. Vorrichtung nach Anspruch 1 oder 2, dadurch gekennzeichnet, daß die untere Fläche (12) des Unterteils (10) an die Form der Resektionsfläche des Knochens angepaßt und/oder an den Knochen andrückbar ist.

4. Vorrichtung nach einem der Ansprüche 1 bis 3, dadurch gekennzeichnet, daß das Unterteil (50; 80) zumindest teilweise in Form eines Dichtrings oder wulstförmig ausgebildet ist.

5. Vorrichtung nach einem der Ansprüche 1 bis 4, dadurch gekennzeichnet, daß das Unterteil (10; 50; 80) und das Oberteil (30; 60; 90) entlang einer möglichst großen Fläche miteinander in Kontakt stehen, wobei diese Fläche gewölbt ist.

6. Vorrichtung nach einem der Ansprüche 1 bis 5, dadurch gekennzeichnet, daß das Unterteil (10) an seiner Unterseite innen eine Ausnehmung (14) aufweist, deren Fläche größer ist als die Markhöhlenöffnung.

7. Vorrichtung nach einem der Ansprüche 1 bis 6, gekennzeichnet durch Einrichtungen (52; 89) zum Verhindern einer Drehbewegung zwischen dem Unterteil (50; 80) und dem Oberteil (60; 90).

8. Vorrichtung nach einem der Ansprüche 1 bis 7, dadurch gekennzeichnet, daß das Unterteil (10; 50; 80) und das Oberteil (30; 60; 90) fest miteinander verbindbar sind.

9. Vorrichtung nach einem der Ansprüche 1 bis 8, dadurch gekennzeichnet, daß das Unterteil (10; 50; 80) aus Silikongummi besteht.

10. Vorrichtung nach einem der Ansprüche 1 bis 9, dadurch gekennzeichnet, daß das Oberteil (30; 60; 90) aus Metall oder Kunststoff, wie TPX® oder einem anderen sterilisierbaren Kunststoff besteht.

## Claims

1. A device for sealing the medullary cavity of a bone during the vacuum application of bone cement, comprising an elastic bottom portion (10; 50; 80) for resting on the bone and for essentially vacuum-tight contact with the bone, and a rigid top portion (30; 60; 90) for pressing the elastic bottom portion against the bone, wherein the top portion is curved upwardly in a shell-like manner and wherein the bottom and top portions comprise a common opening for applying the bone cement.

2. The device according to claim 1, characterized in that the bottom portion (10; 50; 80) and the top portion (30; 60; 90) are essentially approximately hollow-cylindrical, annular, semicircular or ellipsoidal in shape, and the interior of the bottom portion and/or the top portion has a bearing surface (16; 62; 86) for positively receiving the tip of a container (40; 75) out of which the bone cement is applied.

3. The device according to claim 1 or 2, characterized in that the lower surface (12) of the bottom portion (10) is adapted to the shape of the resection plane of the bone and/or can be pressed against the bone.

4. The device according to any of claims 1 to 3, characterized in that at least part of the bottom portion (50; 80) has the shape of a packing ring or a bead.

5. The device according to any of claims 1 to 4, characterized in that the bottom portion (10; 50; 80) and the top portion (30; 60; 90) are in contact with one another over as large an area as possible, said area being curved.

6. The device according to any of claims 1 to 5, characterized in that the interior of the underside of the bottom portion (10) comprises a recess (14) whose surface is larger than the opening of the medullary cavity.

7. The device according to any of claims 1 to 6, having means (52; 89) to prevent a twisting motion between the bottom portion (50; 80) and the top portion (60; 90).

8. The device according to any of claims 1 to 7, characterized in that the bottom portion (10; 50; 80) and the top portion (30; 60; 90) are firmly connectable.

9. The device according to any of claims 1 to 8, characterized in that the bottom portion (10; 50; 80) is made of silicone rubber.

10. The device according to any of claims 1 to 9, characterized in that the top portion (30; 60; 90) is made of metal or plastic such as TPX® or another sterilizable plastic material.

## Revendications

1. Dispositif d'étanchéification de la cavité médullaire d'un os lors de l'application sous vide de ciment pour os, comprenant une partie inférieure élastique (10; 50; 80) pour l'application sur l'os et un contact d'une manière pratiquement étanche au vide avec l'os et une partie supérieure rigide (30; 60; 90) pour le pressage de la partie inférieure élastique sur l'os, la partie supérieure étant cintrée vers le haut en forme de coque, la partie inférieure et la partie supérieure présentant une ouverture commune pour l'application du ciment pour os.

2. Dispositif suivant la revendication 1, caractérisé en ce que la partie inférieure (10; 50; 80) et la partie supérieure (30; 60; 90) sont sensiblement réalisées sous une forme approximativement de cylindre creux, annulaire, hémisphérique ou d'ellipsoïde et en ce que la partie inférieure et/ou la partie supérieure présentent sur leur côté interne une surface d'appui (16, 62; 86) pour recevoir par une liaison de forme la pointe d'un récipient (40; 75) à partir duquel le ciment pour os est appliqué.

3. Dispositif suivant l'une des revendications 1 et 2, caractérisé en ce que la surface inférieure (12) de la partie inférieure (10) est adaptée à la forme de la surface de résection de l'os et/ou est pressable sur l'os.

4. Dispositif suivant l'une des revendications 1 à 3, caractérisé en ce que la partie inférieure (50; 80) est réalisée au moins partiellement sous la forme d'un anneau d'étanchéité ou d'un bourrelet.

5. Dispositif suivant l'une des revendications 1 à 4, caractérisé en ce que la partie inférieure (10; 50; 80) et la partie supérieure (30; 60; 90) sont en contact mutuel le long d'une surface aussi grande que possible, cette surface étant cintrée.

6. Dispositif suivant l'une des revendications 1 à 5, caractérisé en ce que la partie inférieure (10) présente à son côté inférieur, à l'intérieur, un évidement (14) dont la surface est plus grande que l'ouverture de la cavité médullaire.

7. Dispositif suivant l'une des revendications 1 à 6, caractérisé par des agencements (52; 89) pour empêcher un mouvement de rotation entre la partie inférieure (50; 80) et la partie supérieure (60; 90).

8. Dispositif suivant l'une des revendications 1 à 7, caractérisé en ce que la partie inférieure (10; 50; 80) et la partie supérieure (30; 60; 90) peuvent être mutuellement reliées de manière fixe.

9. Dispositif suivant l'une des revendications 1 à 8, caractérisé en ce que la partie inférieure (10; 50; 80) est constituée de caoutchouc de silicone.

10. Dispositif suivant l'une des revendications 1 à 9, caractérisé en ce que la partie supérieure (30; 60; 90) est constituée de métal ou de matière synthétique, comme du TPX® ou une autre matière synthétique stérilisable.
